# EUROPEAN PATENT APPLICATION

(11) **EP 4 226 866 A1**
(43) Date of publication of application: **16.08.2023**
(21) Application number: 21889248.7
(22) Date of filing: 04.11.2021
(51) Int. Cl.: A61B 10/00, F24F 11/63, A61B 5/00, A61B 5/0245, A61B 5/0531, A61B 5/16, A61B 5/352

(54) **DEVICE FOR EVALUATING BODY TEMPERATURE REGULATING FUNCTION, AIR PROCESSING DEVICE, AND METHOD FOR EVALUATING BODY TEMPERATURE REGULATING FUNCTION**

(30) Priority: 04.11.2020 JP 2020184686; 04.11.2020 JP 2020184687
(71) Applicant: Daikin Industries, Ltd., Osaka-shi, Osaka 530-0001 (JP); RIKEN, Wako-shi, Saitama 351-0198 (JP)
(72) Inventor: EMOTO, Shiori, Osaka-shi, Osaka 530-0001 (JP); HORI, Shouta, Osaka-shi, Osaka 530-0001 (JP); SAKATA, Youko, Osaka-shi, Osaka 530-0001 (JP); IWASAKI, Miho, Wako-shi, Saitama 351-0198 (JP); MORITO, Yusuke, Wako-shi, Saitama 351-0198 (JP); WATANABE, Yasuyoshi, Wako-shi, Saitama 351-0198 (JP); MIZUNO, Kei, Wako-shi, Saitama 351-0198 (JP)
(74) Representative: Hoffmann Eitle
(86) International application number: PCT/JP2021/040656
(87) International publication number: WO 2022/097696

(57) **Abstract**

The method for evaluating the body temperature regulating function includes: acquiring an autonomic index of a target; acquiring a perspiration index of the target; and evaluating the body temperature regulating function of the target by using the autonomic index of the target and the perspiration index of the target. In one preferred embodiment, the autonomic index includes at least one of a heart rate variability index or a pulse rate variability index, and the perspiration index includes at least one of a skin conductance change or an amount of perspiration.

## Description

### TECHNICAL FIELD

The present disclosure relates to a device for evaluating a body temperature regulating function, an air processing device, and a method for evaluating a body temperature regulating function.

### BACKGROUND ART

A risk determination device described in Patent Document 1 includes a measurer configured to measure a physiological index associated with the peripheral blood flow of a subject, and an analyzer configuerd to acquire the magnitude of fluctuations in the physiological index (fluctuations in the blood flow) and to determine the risk of abnormality in the thermoregulation function based on the magnitude of the fluctuations.

### CITATION LIST

### PATENT DOCUMENT

Patent Document 1: Japanese Unexamined Patent Publication No. 2011-212306

### SUMMARY OF THE INVENTION

### TECHNICAL PROBLEM

When fluctuations in the blood flow are at or below a certain level, the risk determination device described in Patent Document 1 determines that there is a risk of abnormality in the thermoregulation function. At that time, the thermoregulation function has already been about to fail, and an abnormality in the thermoregulation function may have already occurred.

It is an object of the present disclosure to output the degree of performance of a thermoregulation function of a target.

### SOLUTION TO THE PROBLEM

A first aspect of the present disclosure is directed to a method for evaluating a body temperature regulating function. The method for evaluating the body temperature regulating function includes: acquiring an autonomic index of a target; acquiring a perspiration index of the target; and evaluating the body temperature regulating function of the target by using the autonomic index of the target and the perspiration index of the target.

According to the first aspect, the degree of performance of the body temperature regulating function of the target can be output.

A second aspect of the present disclosure is an embodiment of the first aspect. In the second aspect, the autonomic index includes at least one of a heart rate variability index or a pulse rate variability index, and the perspiration index includes at least one of a skin conductance change or an amount of perspiration.

According to the second aspect, the body temperature regulating function of the target can be evaluated by using the heart rate variability index and the skin conductance change or the amount of perspiration.

A third aspect of the present disclosure is an embodiment of the first or second aspect. In the third aspect, in the evaluating, the body temperature regulating function of the target is evaluated by further using classification information (Z) obtained by classifying a result of evaluating the body temperature regulating function into a plurality of patterns based on a threshold value set for each of the autonomic index and the perspiration index.

According to the third aspect, the body temperature regulating function of the target can be evaluated by using the classification information.

A fourth aspect of the present disclosure is an embodiment of the third aspect. In the fourth aspect, the plurality of patterns are associated with a plurality of index sets, respectively, each of the plurality of index sets is configured as a combination of the autonomic index and the perspiration index each having a range defined by the threshold value, and in the evaluating, one of the plurality of index sets having a range including the autonomic index of the target and the perspiration index of the target is selected, and one of the plurality of patterns associated with the selected index set is evaluated to correspond to the body temperature regulating function of the target.

According to the fourth aspect, the body temperature regulating function of the target can be determined from among the plurality of patterns.

A fifth aspect of the present disclosure is an embodiment of the third or fourth aspect. In the fifth aspect, the threshold value or values set for the autonomic index and/or the perspiration index is/are set for each of different temperatures.

According to the fifth aspect, in consideration of the human physiological phenomenon where the autonomic state and the amount of perspiration vary among different temperatures, the threshold value or values of the autonomic index and/or the perspiration index can be set.

A sixth aspect of the present disclosure is an embodiment of the third or fourth aspect. In the sixth aspect, the threshold value set for each of the autonomic index and the perspiration index is set based on an environment index that represents an environment where the target is situated immediately before the body temperature regulating function is evaluated.

According to the sixth aspect, the threshold value of each of the autonomic index and the perspiration index can be set based on the environment index.

A seventh aspect of the present disclosure is an embodiment of the third or fourth aspect. In the seventh aspect, the threshold value set for each of the autonomic index and the perspiration index is set in consideration of characteristics of the target about an air temperature.

According to the seventh aspect, the threshold value of each of the autonomic index and the perspiration index can be set in consideration of the characteristics of the target about the air temperature.

An eighth aspect of the present disclosure is an embodiment of any one of the third to seventh aspects. In the eighth aspect, the method further includes: making notification of predetermined information to outside if a pattern of the target evaluated in the evaluating corresponds to a predetermined one of the plurality of patterns, and a state where the target corresponds to the predetermined pattern has continued for a predetermined period.

According to the eighth aspect, notification can be made that a state where the target corresponds to the predetermined pattern has continued for a predetermined period.

A ninth aspect of the present disclosure is an embodiment of any one of the first to eighth aspects. In the ninth aspect, the method further includes: controlling a temperature in a room where the target is present, through an air processing device (400), based on an evaluation result in the evaluating.

The ninth aspect allows an action of the air processing device to be controlled in consideration of the result of evaluating the body temperature regulating function of the target.

A tenth aspect of the present disclosure is an embodiment of the ninth aspect. In the tenth aspect, the controlling includes: making the air processing device (400) operate so that the temperature in the room is equal to a predetermined first temperature, if the body temperature regulating function of the target is evaluated to fall under a first pattern; and making the air processing device (400) operate so that the temperature in the room decreases to a predetermined second temperature lower than the predetermined first temperature, and then increases to the predetermined first temperature, if the body temperature regulating function of the target is evaluated to fall under a second pattern that is different from the first pattern.

According to the tenth aspect, control of the air processing device can be configured in accordance with the pattern of the result of evaluating the body temperature regulating function of the target.

An eleventh aspect of the present disclosure is an embodiment of the ninth aspect. In the eleventh aspect, the controlling includes: making the air processing device (400) operate so that the temperature in the room is equal to a predetermined first temperature, if the body temperature regulating function of the target is evaluated to fall under a first pattern; and making the air processing device (400) operate so that the temperature in the room decreases to a predetermined second temperature lower than the predetermined first temperature, and then increases to the predetermined first temperature, if the body temperature regulating function of the target is evaluated to fall under a second pattern that is different from the first pattern and the temperature in the room is higher than or equal to a predetermined temperature.

According to the eleventh aspect, control of the air processing device can be configured in accordance with the pattern of the result of evaluating the body temperature regulating function of the target.

A twelfth aspect of the present disclosure is an embodiment of any one of the first to eleventh aspects. In the twelfth aspect, the method further includes: acquiring a degree of fatigue of a thermoregulation function indicating a degree of fatigue of the body temperature regulating function of the target; and evaluating exercise performance of the target using the degree of fatigue of the thermoregulation function of the target.

In the twelfth aspect, the exercise performance of the target can be evaluated using an index related to the thermoregulation function of the target.

A thirteenth aspect of the present disclosure is an embodiment of the twelfth aspect. In the thirteenth aspect, in the evaluating of the exercise performance, evaluating the exercise performance of the target allows an exercise performance index indicating the exercise performance of the target to be output.

According to the thirteenth aspect, the exercise performance index allows the exercise performance of the target to be checked.

A fourteenth aspect of the present disclosure is an embodiment of the twelfth or thirteenth aspect. In the fourteenth aspect, the acquiring of the index of fatigue includes: acquiring of the autonomic index of the target; the acquiring of the perspiration index of the target.

According to the fourteenth aspect, the exercise performance of the target can be evaluated using the autonomic index and perspiration index of the target.

A fifteenth aspect of the present disclosure is an embodiment of the fourteenth aspect. In the fifteenth aspect, the autonomic index includes a heart rate variability index, and the perspiration index includes a skin conductance change or an amount of perspiration.

According to the fifteenth aspect, the exercise performance of the target can be evaluated using the heart rate variability index and skin conductance change or amount of perspiration of the target.

A sixteenth aspect of the present disclosure is an embodiment of any one of the twelfth to fifteenth aspects. In the sixteenth aspect, in the evaluating of the exercise performance, the exercise performance of the target is evaluated by further using association information (Y) indicating association between each of different values of the degree of fatigue of the thermoregulation function and an exercise performance index indicating the exercise performance.

According to the sixteenth aspect, the exercise performance of the target can be evaluated using the association information (Y).

A seventeenth aspect of the present disclosure is an embodiment of the sixteenth aspect. In the seventeenth aspect, the association information (Y1) includes a plurality of types of information (Y1-1, Y1-2) set according to types of exercise.

The seventeenth aspect allows the exercise performance of the target to be effectively evaluated in accordance with the exercise to be performed by the target.

An eighteenth aspect of the present disclosure is an embodiment of the sixteenth aspect. In the eighteenth aspect, the association information (Y2) includes a plurality of types of information set according to risks related to the thermoregulation function during an exercise.

The eighteenth aspect allows the exercise performance of the target to be effectively evaluated as the risk related to the thermoregulation of the target during an exercise.

A nineteenth aspect of the present disclosure is an embodiment of any one of the twelfth to eighteenth aspects. In the nineteenth aspect, the method further includes: making notification of information related to a result of evaluating exercise performance of the target.

According to the nineteenth aspect, the target can check his/her own exercise performance.

A twentieth aspect of the present disclosure is an embodiment of any one of the twelfth to nineteenth aspects. In the twentieth aspect, the method further includes at least one of: making an air processing device (400) control a temperature, a humidity, and/or an airflow in a space where the target is present, based on an evaluation result in the evaluating of the exercise performance; or making a temperature processing device control a temperature, a humidity, and/or an airflow of an appliance or clothing touched by the target.

The twentieth aspect allows an action of the air processing device to be controlled in consideration of the result of evaluating the exercise performance of the target.

A twenty-first aspect of the present disclosure is directed to a device for evaluating a body temperature regulating function. The device for evaluating the body temperature regulating function includes: a first acquisition unit (310) configured to acquire an autonomic index of a target; a second acquisition unit (320) configured to acquire a perspiration index; and an evaluation unit (350) configured to evaluate the body temperature regulating function of the target by using the autonomic index of the target and the perspiration index of the target.

According to the twenty-first aspect, the degree of performance of the body temperature regulating function of the target can be output.

A twenty-second aspect of the present disclosure is directed to an air processing device. The air processing device includes: the device (300) for evaluating the body temperature regulating function.

The twenty-second aspect allows an action of the air processing device to be controlled in consideration of the result obtained by the evaluation device for the body temperature regulating function evaluating the body temperature regulating function of the target.

### BRIEF DESCRIPTION OF THE DRAWINGS

FIG. 1 is a block diagram illustrating a configuration of a system for evaluating a body temperature regulating function according to a first embodiment of the present invention.
FIG. 2 shows a graph plotted on a coordinate system with the vertical axis representing a heart rate variability index and the horizontal axis representing time.
FIG. 3 shows a graph plotted on a coordinate system with the vertical axis representing skin conductance change and the horizontal axis representing time.
FIG. 4 shows classification information.
FIG. 5 is a block diagram illustrating a configuration of a system for evaluating a body temperature regulating function according to a second embodiment of the present invention.
FIG. 6 is a flowchart showing an example action of an air processing device.
FIG. 7 shows the skin conductance change and the indoor temperature when a first process is performed.
FIG. 8 shows the skin conductance change and the indoor temperature when a second process is performed.
FIG. 9 shows environment-associated information indicating association among an environment index, a first threshold value, and a second threshold value.
FIG. 10 is a block diagram illustrating a configuration of a system for evaluating exercise performance according to a third embodiment of the present invention.
FIG. 11 shows a correlation model between the degree of fatigue of the body temperature regulating function and the exercise performance.
FIG. 12 shows first association information.
FIG. 13 is a flowchart showing an example of processing of an evaluation unit.
FIG. 14 shows second association information.
FIG. 15 is a block diagram illustrating a configuration of a system for evaluating exercise performance according to a fifth embodiment of the present invention.

### DESCRIPTION OF EMBODIMENTS

Embodiments of the present invention will be described in detail with reference to the drawings. Note that like reference characters denote the same or equivalent components in the drawings, and the detailed description thereof, the description of advantages associated therewith, and other descriptions will not be repeated.

### -First Embodiment-

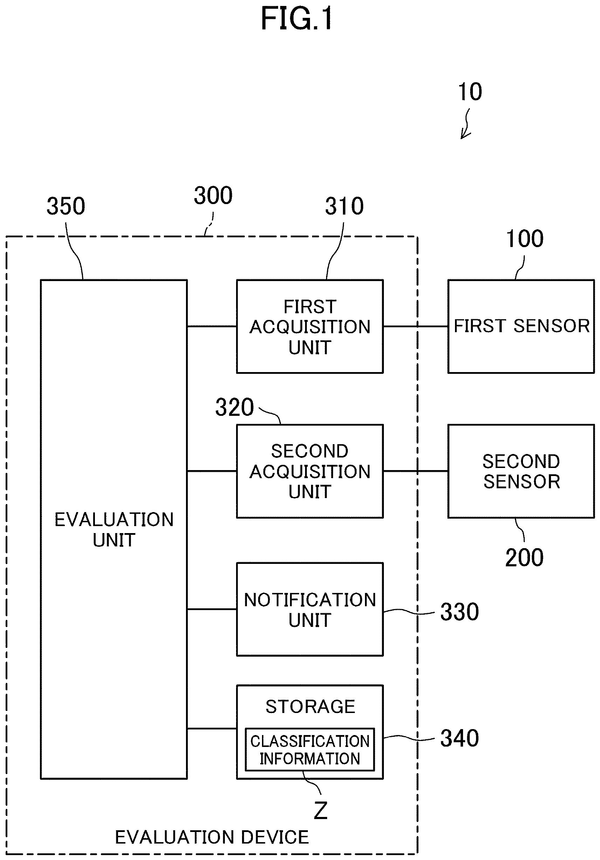

A system (10) for evaluating a body temperature regulating function according to a first embodiment of the present invention will be described with reference to FIG. 1. FIG. 1 is a block diagram illustrating a configuration of the system (10) for evaluating the body temperature regulating function according to the first embodiment of the present invention. The system (10) for evaluating the body temperature regulating function may be hereinafter referred to as the "evaluation system (10)."

### -General Configuration-

As shown in FIG. 1, the evaluation system (10) includes a first sensor (100), a second sensor (200), and a device (300) for evaluating the body temperature regulating function. The device (300) for evaluating the body temperature regulating function may be hereinafter referred to as the "evaluation device (300)."

The first sensor (100) is worn by a target to measure the physical quantity indicating how a control system related to the autonomic nervous system of the target works. In the first embodiment, the first sensor (100) measures the heart rate of the target, which is an example of the physical quantity. The first sensor (100) includes, for example, an electrocardiographic sensor.

The second sensor (200) is worn by the target to measure the perspiration index of the target. The perspiration index indicates the physical quantity that changes according to perspiration on the skin surface. Examples of the perspiration index include electrodermal activity (EDA), such as skin potential activity (SPA) or skin conductance change (SC), and the amount of perspiration. In the first embodiment, the skin conductance change (SC) is employed as the perspiration index. In this case, the second sensor (200) includes a skin conductance change measuring device, and measures the skin conductance change (SC) of the target. Note that the amount of perspiration may be employed as the perspiration index. In this case, the second sensor (200) includes an amount-of-perspiration determining device, such as a perspiration meter, and measures the amount of perspiration of the target.

The evaluation device (300) includes, for example, a terminal, such as a smartphone or a personal computer (PC). The evaluation device (300) includes a first acquisition unit (310), a second acquisition unit (320), a notification unit (330), a storage (340), and an evaluation unit (350). The evaluation device (300) evaluates the body temperature regulating function of the target to output information indicating the degree of performance of the body temperature regulating function of the target. The body temperature regulating function indicates the function of maintaining the body temperature through heat dissipation (e.g., perspiration) or thermogenesis (e.g., body shivering) as appropriate to address heat stress. The heat stress indicates the load (e.g., warm and cold stimuli that hinder maintenance of the body temperature) that a human receives from the thermal environment. The degree of performance of the body temperature regulating function is defined by how often an action for thermoregulation, such as an action of perspiration, an action of body shivering, or an action of dilating or constricting the blood vessels, is performed in response to an instruction related to the thermoregulation function through the autonomic nerve from the center to the terminals.

The first acquisition unit (310) includes a device for communicating with the first sensor (100). The first acquisition unit (310) acquires information indicating the interbeat interval measured by the first sensor (100). The first acquisition unit (310) is communicably connected to the first sensor (100) in a wired or wireless manner. The first acquisition unit (310) includes, for example, at least one of a device for performing wireless communication (e.g., Bluetooth (registered trademark), wireless fidelity (Wi-Fi) (registered trademark), or Internet communications) (such as a wireless LAN module) or a device for performing wired communication (such as a communication port connected to a communication cable).

The first acquisition unit (310) further includes a processor. The processor of the first acquisition unit (310) calculates an autonomic nerve index based on the measurement result of the first sensor (100). The autonomic nerve index indicates an index used to grasp how the control system related to the autonomic nervous system works. In the first embodiment, the autonomic nerve index indicates a heart rate variability index (LF/HF) used to grasp how heartbeat works. The processor of the first acquisition unit (310) calculates the heart rate variability index (LF/HF) based on the heart rate, which is the measurement result of the first sensor (100). The heart rate variability index (LF/HF) can be calculated from the interbeat interval by frequency analysis, for example. Specifically, frequency analysis (spectrum analysis) performed on the heart rate variability (R-R interval variability) using a technique, such as Fourier transformation or wavelet transformation, enables acquisition of a low-frequency component (LF), a high-frequency component (HF), and the ratio of the low-frequency component to the high-frequency component (LF/HF). The low-frequency component (LF) mainly reflects the sympathetic nerve function (partially reflects the parasympathetic nerve function), and ranges from 0.05 Hz to 0.15 Hz. The high-frequency component (HF) reflects the parasympathetic nerve function, and ranges from 0.15 Hz to 0.4 Hz. The ratio is the heart rate variability index. Alternatively, the waveform of a pulse wave may undergo secondary differentiation to calculate an acceleration plethysmogram. The variability of the a-a intervals (pulse periods) corresponding to the variability of the R-R intervals of an electrocardiogram may be determined based on the waveform of the obtained acceleration plethysmogram. Frequency analysis may be performed on the time variability of the a-a intervals. Accordingly, the heart rate variability index (LF/HF) can be determined based on the result of the analysis.

In the first embodiment, the first acquisition unit (310) performs the process of calculating the heart rate variability index (LF/HF). However, the present invention is not limited to this. The first sensor (100) may be configured to perform the process of calculating the heart rate variability index (LF/HF), and the first acquisition unit (310) may be configured to acquire the heart rate variability index (LF/HF) from the first sensor (100).

The second acquisition unit (320) includes a device for communicating with the second sensor (200). The second acquisition unit (320) acquires information indicating the skin conductance change (SC) measured by the second sensor (200). The second acquisition unit (320) is communicably connected to the second sensor (200) in a wired or wireless manner. The second sensor (200) includes, for example, at least one of a device for performing wireless communication or a device for performing wired communication.

The notification unit (330) makes notification of predetermined information. The notification unit (330) includes, for example, a speaker that emits notification sounds, a display that displays predetermined information, and/or a communication device that transmits predetermined information to an external terminal (such as a smartphone). The predetermined information will be described later.

The storage (340) includes a main memory (e.g., a semiconductor memory), such as a flash memory, a read only memory (ROM), and a random access memory (RAM), and may further include an auxiliary memory (e.g., a hard disk drive, a solid state drive (SSD), a secure digital (SD) memory card, or a universal serial bus (USB) flash memory). The storage (340) stores various computer programs executed by the evaluation unit (350) and the processor of the first acquisition unit (310).

The storage (340) stores the classification information (Z). The classification information (Z) will be described later.

The evaluation unit (350) includes a processor, such as a central processing unit (CPU) or a microprocessor unit (MPU). The evaluation unit (350) executes a computer program stored in the storage (340) so as to control elements of the evaluation device (300).

### -Description of Test Result-

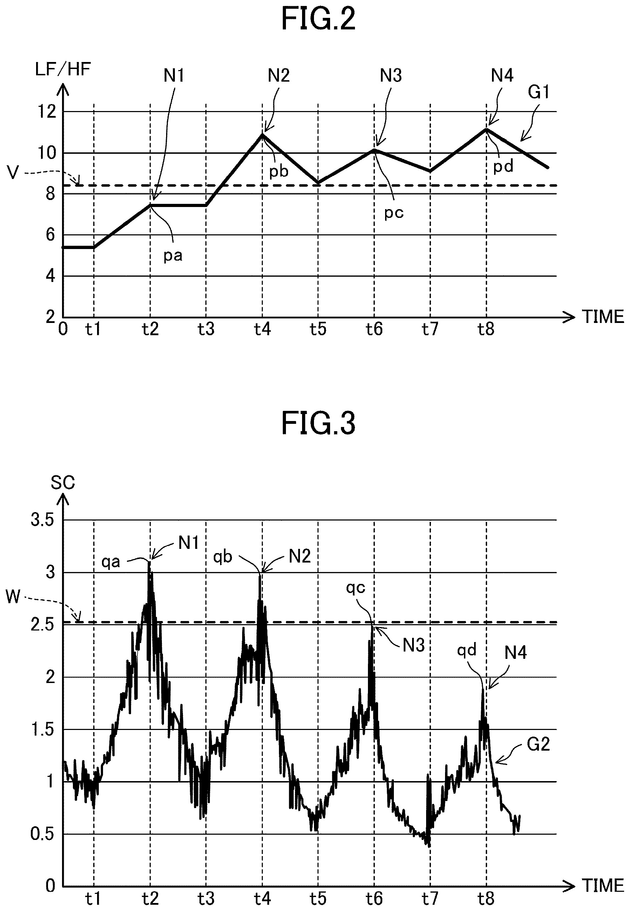

The result of the test performed by the inventors of this application will be described with reference to FIGS. 2 and 3.

FIG. 2 shows a graph (G1) plotted on a coordinate system with the vertical axis representing the heart rate variability index (LF/HF) and the horizontal axis representing time. In general, as the heart rate variability index (LF/HF) increases, the stress increases.

FIG. 3 shows a graph (G2) plotted on a coordinate system with the vertical axis representing the skin conductance change (SC) and the horizontal axis representing time. Each of the graphs (G1) and (G2) shows the result of this test. In general, as the skin conductance change (SC) increases, the amount of perspiration increases.

In this test, the target moved alternately to two rooms. The air temperature in one of the two rooms was set at a room temperature (26°C), and the air temperature in the other room was set at a high temperature (36°C). In addition, the heart rate variability index (LF/HF) and skin conductance change (SC) of the target were determined at all times during the test period. Then, the graph (G1) in which the time for which the test has been performed is associated with the heart rate variability index (LF/HF) of the target (see FIG. 2) is created. The graph (G2) in which the time for which the test has been performed is associated with the skin conductance change (SC) of the target (see FIG. 3) is created.

As shown in FIGS. 2 and 3, in this test, the periods from time 0 to time 11, from time t2 to time t3, from time t4 to time t5, from time t6 to time t7, and after time t8 are 26°C room-temperature periods. The periods from time t1 to time t2, from time t3 to time t4, from time t5 to time t6, and from time t7 to time t8 are 36°C high-temperature periods.

The graph (G1) will be described with reference to FIG. 2.

As shown in the graph (G1), during the room-temperature periods, the heart rate variability index (LF/HF) of the target tends to remain unchanged or to decrease. During the high-temperature periods, the heart rate variability index (LF/HF) of the target tends to increase.

The graph (G2) will be described with reference to FIG. 3.

As shown in the graph (G2), during the room-temperature periods, the skin conductance change (SC) of the target tends to decrease. During the high-temperature periods, the skin conductance change (SC) of the target tends to increase.

The relation between the heart rate variability index (LF/HF) and the skin conductance change (SC) will be described with reference to FIGS. 2 and 3.

As shown in FIGS. 2 and 3, during the first high-temperature period (from time t1 to time t2), the peak value (pa) of the heart rate variability index (LF/HF) is lower and the peak value (qa) of the skin conductance change (SC) is higher than during the other high-temperature periods. That is, even if the activity of the sympathetic nerve that is responsible for the perspiration function is not excessive, an adequate amount of perspiration is observed. Thus, it can be understood that while the load imposed on the thermoregulation function is high, the body temperature has been regulated with enough energy left over.

During the second high-temperature period (from time t3 to time t4), the peak value (pb) of the heart rate variability index (LF/HF) is higher than the peak value (pa) during the first high-temperature period (pb > pa). Thus, it is conceivable that a balance is starting to be disturbed between the sympathetic nerve that is responsible for the thermoregulation function and the parasympathetic nerve that has antagonism with the thermoregulation function. Meanwhile, the peak value (qb) of the skin conductance change (SC) is equivalent to the peak value (qa) during the first high-temperature period (qb ≈ qa). Thus, it is conceivable that the thermoregulation function is maintained. Thus, it can be understood that while continuation of the load imposed on the thermoregulation function reduces the amount of energy left over, the thermoregulation function has been maintained.

During the third high-temperature period (from time t5 to time t6), the peak value (pc) of the heart rate variability index (LF/HF) is high just like during the second high-temperature period, whereas the peak value (qc) of the skin conductance change (SC) is lower than the peak value (qb) during the second high-temperature period. Thus, it can be expected that the disturbance of the autonomic balance and the exhaustion of a peripheral physiological mechanism related to perspiration have prevented adequate perspiration. It can be understood that the thermoregulation function in this state is more exhausted than during the second high-temperature period.

During the fourth high-temperature period (from time t7 to time t8), the peak value (pd) of the heart rate variability index (LF/HF) is high just like during the second and third high-temperature periods, whereas the peak value (qd) of the skin conductance change (SC) is further lower than the peak value (qc) during the third high-temperature period. This leads to a further decrease in the amount of perspiration of the target. Thus, it can be understood that the body temperature regulating function is further exhausted, which results in difficulty in thermoregulation.

The above-described test has provided the result that if the target stays alternately in a hot environment (36°C) and in a room-temperature environment (26°C) each for 20 minutes, his/her index related to the thermoregulation function is gradually made worse.

Based on the above-described test result, the inventors of this application have developed a technique for evaluating the fatigued state of the thermoregulation function (the state of the thermoregulation function), such as the disturbance of the autonomic balance related to the temperature regulating function or deterioration in the peripheral perspiration function, step by step based on the heart rate variability index (LF/HF) and the state of the skin conductance change serving as the perspiration index. The fatigued state is caused by long-time or repetitive exposure to heat load.

### -Classification Information-

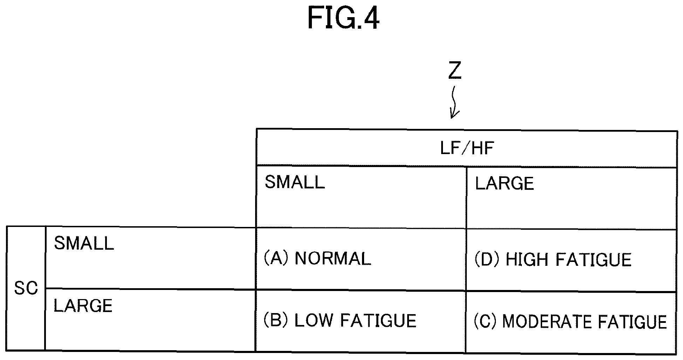

The classification information (Z) stored in the storage (340) will be described with reference to FIGS. 1 to 4. FIG. 4 shows the classification information (Z).

The classification information (Z) indicates that the result of evaluating the body temperature regulating function is classified into a plurality of patterns based on the threshold values respectively set for the autonomic index and the perspiration index. The plurality of patterns are associated with a plurality of index sets, respectively. The plurality of index sets are each configured as a combination of the autonomic index and the perspiration index each having a range defined by the associated threshold value.

The threshold value set for the autonomic index and the threshold value set for the perspiration index may be hereinafter referred to as the "first threshold value" and the "second threshold value," respectively.

In the first embodiment, the classification information (Z) is obtained by classifying the result of evaluating the body temperature regulating function into a plurality of patterns using the heart rate variability index (LF/HF), which is an example of the autonomic index, and the skin conductance change (SC), which is an example of the perspiration index.

In the first embodiment, the result of evaluating the body temperature regulating function is classified into four patterns (A) to (D). The pattern (A) indicates the normal state where the load imposed on the thermoregulation is low and the thermoregulation function has not been fatigued. The pattern (B) indicates the state where while the load imposed on the thermoregulation is high, the fatigue of the thermoregulation function is low such that energy is still left over. The pattern (C) indicates the state where the load imposed on the thermoregulation is high and the fatigue of the thermoregulation function is moderate such that a small amount of energy is left over. The pattern (D) indicates the state where the load imposed on the thermoregulation is high and the fatigue of the thermoregulation function is high such that the body temperature is hardly maintained. The result of evaluating the body temperature regulating function becomes worse in the order of the patterns (A), (B), (C), and (D).

In the first embodiment, a first threshold value V is set for the heart rate variability index (LF/HF) (see FIG. 2). A second threshold value W is set for the skin conductance change (SC) (see FIG. 3).

In the classification information (Z) shown in FIG. 4, the term "large" described in the space for the heart rate variability index (LF/HF) indicates that the heart rate variability index (LF/HF) acquired by the first acquisition unit (310) is greater than or equal to the first threshold value V The term "small" described in the space for the heart rate variability index (LF/HF) indicates that the heart rate variability index (LF/HF) acquired by the first acquisition unit (310) is less than the first threshold value V The term "large" described in the space for the skin conductance change (SC) indicates that the skin conductance change (SC) acquired by the second acquisition unit (320) is greater than or equal to the second threshold value W. The term "small" described in the space for the skin conductance change (SC) indicates that the skin conductance change (SC) acquired by the second acquisition unit (320) is less than the second threshold value W.

In the first embodiment, the pattern (A) is configured as a combination of the heart rate variability index (LF/HF) having a range less than the first threshold value V and the skin conductance change (SC) having a range less than the second threshold value W. As indicated by the pattern (A), if the heart rate variability index (LF/HF) is less than the first threshold value V, and the skin conductance change (SC) is less than the second threshold value W, this state can be evaluated as the normal state where the load imposed on the thermoregulation is low and the thermoregulation function has not been fatigued.

In the first embodiment, the pattern (B) is configured as a combination of the heart rate variability index (LF/HF) having a range less than the first threshold value V and the skin conductance change (SC) having a range greater than or equal to the second threshold value W. As indicated by the pattern (B), if the heart rate variability index (LF/HF) is less than the first threshold value V, and the skin conductance change (SC) is greater than or equal to the second threshold value W, the autonomic nervous system acts normally, and a large amount of perspiration occurs. Thus, this state can be evaluated as the state where while the load imposed on the thermoregulation is high, the fatigue of the thermoregulation function is low such that energy is still left over.

In the first embodiment, the pattern (C) is configured as a combination of the heart rate variability index (LF/HF) having a range greater than or equal to the first threshold value V and the skin conductance change (SC) having a range greater than or equal to the second threshold value W. As indicated by the pattern (C), if the heart rate variability index (LF/HF) is greater than or equal to the first threshold value V, and the skin conductance change (SC) is greater than or equal to the second threshold value W, the autonomic nerve that is responsible for the thermoregulation function works excessively, and a large amount of perspiration occurs. Thus, this state can be evaluated as the state where the load imposed on the thermoregulation is high and the fatigue of the thermoregulation function is moderate so that a small amount of energy is left over.

In the first embodiment, the pattern (D) is configured as a combination of the heart rate variability index (LF/HF) having a range greater than or equal to the first threshold value V and the skin conductance change (SC) having a range less than the second threshold value W. As indicated by the pattern (D), if the heart rate variability index (LF/HF) is greater than or equal to the first threshold value V, and the skin conductance change (SC) is less than the second threshold value W, the autonomic nerve that is responsible for the thermoregulation function works excessively, whereas the perspiration function that responds to the autonomic nerve deteriorates. Thus, this state can be evaluated as the state where the load imposed on the thermoregulation function is high and the fatigue of the thermoregulation function is high such that the body temperature is hardly maintained.

### -Example of Processing of Evaluation Unit (350)-

An example of processing of the evaluation unit (350) will be described with reference to FIGS. 2 to 4.

As shown in FIGS. 2 to 4, in the first embodiment, at first timing (N1) around the end of the first high-temperature period (from time t1 to time t2), the heart rate variability index (LF/HF) reaches its peak value (pa), and the skin conductance change (SC) reaches its peak value (qa). At the first timing (N1), the heart rate variability index (LF/HF = pa) is less than the first threshold value V, and the skin conductance change (SC = qa) is greater than or equal to the second threshold value W. In this case, the evaluation unit (350) (see FIG. 1) evaluates that the body temperature regulating function of the target corresponds to the pattern (B).

In the first embodiment, at second timing (N2) around the end of the second high-temperature period (from time t3 to time t4), the heart rate variability index (LF/HF) reaches its peak value (pb), and the skin conductance change (SC) reaches its peak value (qb). At the second timing (N2), the heart rate variability index (LF/HF = pb) is greater than or equal to the first threshold value V, and the skin conductance change (SC = qb) is greater than or equal to the second threshold value W. In this case, the evaluation unit (350) (see FIG. 1) evaluates that the body temperature regulating function of the target corresponds to the pattern (C).

In the first embodiment, at third timing (N3) around the end of the third high-temperature period (from time t5 to time t6), the heart rate variability index (LF/HF) reaches its peak value (pc), and the skin conductance change (SC) reaches its peak value (qc). At the third timing (N3), the heart rate variability index (LF/HF = pc) is greater than or equal to the first threshold value V, and the skin conductance change (SC = qc) is less than the second threshold value W. In this case, the evaluation unit (350) (see FIG. 1) evaluates that the body temperature regulating function of the target corresponds to the pattern (D).

In the first embodiment, at fourth timing (N4) around the end of the fourth high-temperature period (from time t7 to time t8), the heart rate variability index (LF/HF) reaches its peak value (pd), and the skin conductance change (SC) reaches its peak value (qd). At the fourth timing (N4), the heart rate variability index (LF/HF = pd) is greater than or equal to the first threshold value V, and the skin conductance change (SC = qc) is less than the second threshold value W. In this case, the evaluation unit (350) (see FIG. 1) evaluates that the body temperature regulating function of the target corresponds to the pattern (D).

### -Advantages of First Embodiment-

As described above with reference to FIGS. 1 to 4, the evaluation unit (350) evaluates the body temperature regulating function of the target using the heart rate variability index (LF/HF) serving as the autonomic index of the target and the skin conductance change (SC) serving as the perspiration index of the target. As a result, information indicating the degree of performance of the thermoregulation function of the target as indicated by the patterns (A) to (D) in FIG. 4 can be output as the result of evaluating the body temperature regulating function. The degree of performance of the body temperature regulating function of the target can be checked, and so an environment suitable for the target can be provided. In addition, the degree of performance of the body temperature regulating function of the target can be checked, and so the temperature or any other element in a space where the target is present can be controlled before exhaustion of the body temperature regulating function of the target so that the environment in the space becomes comfortable for the target.

### -Second Embodiment-

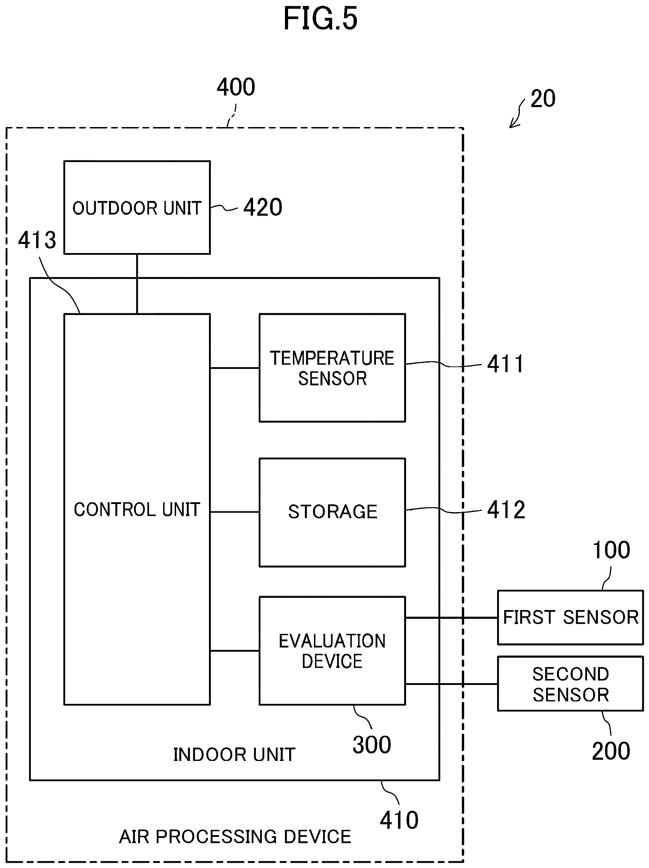

An evaluation system (20) according to a second embodiment of the present invention will be described with reference to FIG. 5. FIG. 5 is a block diagram illustrating a configuration of the evaluation system (20) according to the second embodiment of the present invention.

### -General Configuration-

As shown in FIG. 5, the evaluation system (20) includes a first sensor (100), a second sensor (200), and an air processing device (400).

The air processing device (400) is an appliance having the function of regulating an indoor temperature. The air processing device (400) include, for example, an air conditioner.

The air processing device (400) includes an indoor unit (410) and an outdoor unit (420).

The indoor unit (410) is placed inside. The indoor unit (410) includes a temperature sensor (411), a storage (412), an evaluation device (300), and a control unit (413). The temperature sensor (411) detects the indoor temperature. The storage (412) includes a main memory, such as a flash memory, a ROM, or a RAM, and may further include an auxiliary memory. The storage (412) stores therein various computer programs executable by the control unit (413). The control unit (413) includes a processor, such as a central processing unit (CPU) or a microprocessor unit (MPU). In the second embodiment, the evaluation device (300) is an electronic component incorporated into or externally attached to a housing of the indoor unit (410).

The control unit (413) executes a computer program stored in the storage (412) so as to control elements of the air processing device (400). The control unit (413) controls components, such as a fan provided in the indoor unit (410) and a compressor provided in the outdoor unit (420), to control air conditioning, thus controlling the indoor temperature. The control unit (413) of the air processing device (400) may function also as the evaluation unit (350) of the evaluation device (300) (see FIG. 1). The storage (412) of the air processing device (400) may function also as the storage (340) of the evaluation device (300).

### -Example of Action of Air Processing Device (400)-

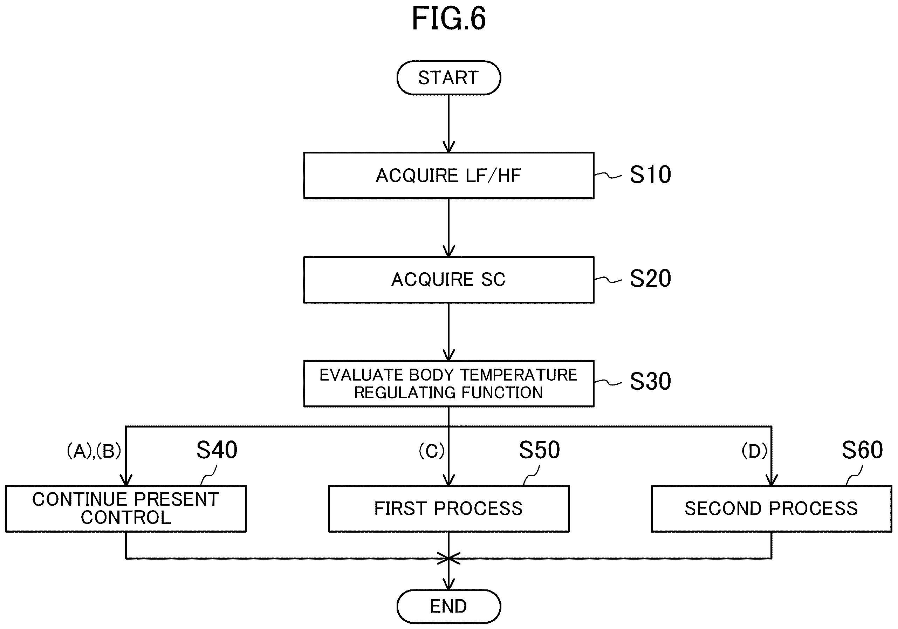

An example action of the air processing device (400) will be described with reference to FIGS. 1 and 5 to 8. FIG. 6 is a flowchart showing an example action of the air processing device (400).

In the second embodiment, the process shown in FIG. 6 is started while the control unit (413) of the air processing device (400) is controlling air conditioning. For example, the control unit (413) of the air processing device (400) performs predicted mean vote (PMV) control. The PMV is an index for quantitatively treating a thermal sensation felt by a human. The PMV control is control intended to maintain the PMV at a value at which a large majority of people feel comfortable.

At the start of the process shown in FIG. 6, for example, the control unit (413) of the air processing device (400) controls air conditioning in a room such that the indoor temperature reaches the start temperature (hot temperature). The start temperature indicates, for example, the indoor temperature when air conditioning is controlled so that the PMV is greater than or equal to +1. The process shown in FIG. 6 is performed while the target is present in the room.

As shown in FIGS. 1, 5 and 6, in step S10, the heart rate of the target is measured by the first sensor (100). The first acquisition unit (310) of the evaluation device (300) acquires the heart rate of the target from the first sensor (100), and calculates the heart rate variability index (LF/HF) of the target based on the acquired heart rate of the target. As a result, the first acquisition unit (310) of the evaluation device (300) acquires the heart rate variability index (LF/HF) of the target.

In step S20, the skin conductance change (SC) of the target is measured by the second sensor (200). The second acquisition unit (320) of the evaluation device (300) acquires the skin conductance change (SC) of the target from the second sensor (200).

In steps S10 and S20, the heart rate variability index (LF/HF) and the skin conductance change (SC) measured at substantially the same time are acquired. Note that the order of the process shown in step S10 and the process shown in step S20 is not limited. For example, step S20 and step S10 may be performed in this order, or may be performed at the same time.

In step S30, the evaluation unit (350) of the evaluation device (300) evaluates the body temperature regulating function of the target based on the heart rate variability index (LF/HF) acquired in step S10, the skin conductance change (SC) acquired in step S20, and the classification information (Z) (see FIG. 4). In the second embodiment, the body temperature regulating function of the target is evaluated to fall under any one of the patterns (A) to (D).

If the body temperature regulating function of the target is evaluated to fall under the pattern (A), the process proceeds to step S40. Also if the body temperature regulating function of the target is evaluated to fall under the pattern (B), the process proceeds to step S40. If the body temperature regulating function of the target is evaluated to fall under the pattern (C) (first pattern), the process proceeds to step S50. If the body temperature regulating function of the target is evaluated to fall under the pattern (D) (second pattern), the process proceeds to step S60.

In step S40, the control unit (413) of the air processing device (400) continues to perform the present air-conditioning control (the air-conditioning control that has been performed since the start of the process shown in FIG. 6). In the second embodiment, the control unit (413) of the air processing device (400) continues the air-conditioning control performed so that the indoor temperature is equal to the start temperature (so that the PMV is greater than or equal to +1). When the process shown in step S40 ends, the process ends.

In step S50, the control unit (413) of the air processing device (400) performs a first process. The first process indicates that air conditioning is controlled so that the indoor temperature is equal to a predetermined first temperature (comfortable temperature). The first temperature indicates, for example, the indoor temperature when air conditioning is controlled so that the PMV is from -0.5 to +0.5. The first temperature is lower than the start temperature.

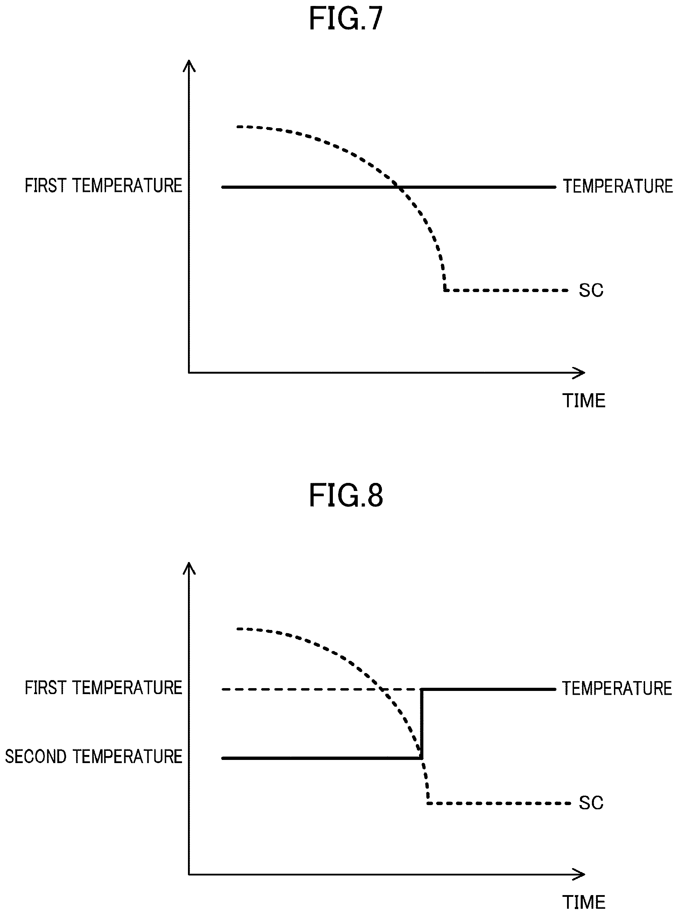

FIG. 7 shows the skin conductance change (SC) and the indoor temperature when first process is performed. As shown in FIG. 7, when the first process is performed to lower the indoor temperature from the start temperature to the first temperature, the amount of perspiration of the target decreases, and the skin conductance change (SC) decreases. Then, stopping of the perspiration of the target causes the skin conductance change (SC) to converge to a fixed value. A state where the skin conductance change (SC) converges to the fixed value continues for a certain period, and so the body temperature regulating function of the target is improved so that the body temperature regulating function of the target is evaluated to fall under the pattern (A) or (B). When the process shown in step S50 ends, the process ends.

As shown in FIGS. 1, 5, and 6, in step S60, the control unit (413) of the air processing device (400) performs a second process. The second process indicates that after a state where the indoor temperature is equal to a predetermined second temperature (cold temperature) has been continued for a predetermined period, air conditioning is controlled so that the indoor temperature is equal to the predetermined first temperature (comfortable temperature). The second temperature indicates, for example, the indoor temperature when air conditioning is controlled so that the PMV is less than -1. The second temperature is lower than the first temperature.

FIG. 8 shows the skin conductance change (SC) when second process is performed. As shown in FIG. 8, performing the second process causes the indoor temperature to decrease from the start temperature to the second temperature for a predetermined period, and then to increase to the first temperature. Offering an environment at the second temperature (cold temperature) to the target quickly reduces the amount of perspiration of the target. Thereafter, an environment at the first temperature (comfortable temperature) can be offered to the target. As a result, the state where the skin conductance change (SC) has converged to the fixed value (the state where the target is substantially prevented from perspiring) can be continued for a certain period. This can improve the body temperature regulating function of the target so that the body temperature regulating function of the target is evaluated to fall under the pattern (A) or (B). The predetermined period (the period during which air conditioning is controlled so that the indoor temperature is equal to the second temperature) indicates, for example, a period until the skin conductance change (SC) becomes equal to or less than a predetermined value, a period until the skin conductance change (SC) converges to the fixed value, or a period until a predetermined time elapses since the skin conductance change (SC) converges to the fixed value. When the process shown in step S60 ends, the process ends.

If the body temperature regulating function of the target deteriorates to the pattern (D), the skin conductance change (SC) is immediately lowered to quickly improve the body temperature regulating function of the target in one preferred embodiment. Thus, in the second process (step S60), the indoor temperature is adjusted to the second temperature (cold temperature) before being adjusted to the first temperature (comfortable temperature), thereby immediately lowering the skin conductance change (SC) of the target. As a result, the body temperature regulating function of the target can be quickly improved.

In the second process, a process similar to the first process (the process of lowering the indoor temperature from the start temperature to the first temperature) may be performed. In the first process (step S50), a process similar to the second process (the process of lowering the indoor temperature from the start temperature to the first temperature and then increasing the indoor temperature to the second temperature) may be performed.

### -Advantages of Second Embodiment-

As described above, the air processing device (400) includes the evaluation device (300). This allows operation of the air processing device (400) to be controlled in consideration of the result obtained by the evaluation device (300) evaluating the body temperature regulating function of the target.

### <<Other Embodiments>>

While the first and second embodiments have been described above, it will be understood that various changes in form and details may be made without departing from the spirit and scope of the claims (e.g., (1) to (10)). The embodiments and the variations thereof may be combined and replaced with each other without deteriorating intended functions of the present disclosure.
(1) In each of the first and second embodiments, the heart rate variability index (LF/HF) used to grasp how the heart rate works is used as the autonomic index. However, the present invention is not limited to this. For example, the pulse rate variability index used to grasp how the pulse rate works may be used as the autonomic index. In this case, the first sensor (100) includes a pulse wave sensor.
(2) If the evaluation unit (350) of the evaluation device (300) evaluates that the body temperature regulating function of the target corresponds to the pattern (D) (predetermined pattern), and further determines that a state where the body temperature regulating function of the target corresponds to the pattern (D) has been continued for a predetermined period, the evaluation unit (350) may be configured to control the notification unit (330) (see FIG. 1) to make notification of predetermined information (information indicating a conclusion generally derived from the determination result) to the outside. In this case, for example, if the state where the body temperature regulating function of the target corresponds to the pattern (D) has been continued for the predetermined period, the evaluation unit (350) of the evaluation device (300) determines that the target has developed heat stroke. Then, the notification unit (330) makes notification of information indicating that the target has developed heat stroke, as an example of the predetermined information. As a result, the evaluation device (300) can be used for measures against heat stroke.
(3) In the second embodiment, air conditioning is controlled using the evaluation result of the evaluation device (300). However, the present invention is not limited to this. For example, the evaluation result of the evaluation device (300) may be used to recommend drinking water.
(4) In each of the first and second embodiments, the first threshold value for sorting the values of the autonomic index (heart rate variability index (LF/HF)) and the second threshold value for sorting the values of the perspiration index (skin conductance change (SC)) are set to respective fixed values (the first threshold value V and the second threshold value W) (see FIGs. 2 and 3). However, the present invention is not limited to this.

The first threshold value may be set for each of different temperatures. The temperature in this case specifically indicates the temperature in the space where the target is present. In this case, for example, if the temperature is 36°C, the first threshold value is set to 9 (= LF/HF), and if the temperature is 31°C, the first threshold value is set to 8.5 (= LF/HF). Thus, in consideration of the human physiological phenomenon where the autonomic state varies among different temperatures, the first threshold value can be set for each temperature so as to be adapted to the autonomic state. As a result, the first threshold value can be more accurately set.

An example device configuration for setting the first threshold value for each temperature will be described with reference to FIG. 1. As illustrated in FIG. 1, in this case, the evaluation system (10) further includes a temperature sensor (not shown) that detects the temperature. The storage (340) stores first association information. The first association information is configured as, for example, a table or a graph in which the first threshold value is associated with each of different temperatures. Acquiring information indicating the temperature from the temperature sensor, the evaluation unit (350) selects one of the first threshold values associated with the acquired temperature in the first association information. The evaluation unit (350) uses the selected first threshold value to sort the values of the heart rate variability index (LF/HF) in the classification information (Z) (see FIG. 4) into a range of larger values and a range of smaller values, and uses the sorted classification information (Z) to evaluate the body temperature regulating function of the target.

The second threshold value may be set for each of different temperatures. The temperature in this case specifically indicates the temperature in the space where the target is present. In this case, for example, if the temperature is 36°C, the second threshold value is set to 2 (= SC), and if the temperature is 31°C, the second threshold value is set to 1.5 (= SC). Thus, in consideration of the human physiological phenomenon where the amount of perspiration varies among different temperatures, the second threshold value can be set for each temperature so as to be adapted to the amount of perspiration. As a result, the second threshold value can be more accurately set.

An example device configuration for setting the second threshold value for each temperature will be described with reference to FIG. 1. As illustrated in FIG. 1, in this case, the evaluation system (10) further includes a temperature sensor (not shown) that detects the temperature. The storage (340) stores second association information. The second association information is configured as, for example, a table or a graph in which the second threshold value is associated with each of different temperatures. Acquiring information indicating the temperature from the temperature sensor, the evaluation unit (350) selects one of the second threshold values associated with the acquired temperature in the second association information. The evaluation unit (350) uses the selected second threshold value to sort the values of the skin conductance change (SC) in the classification information (Z) (see FIG. 4) into a range of larger values and a range of smaller values, and uses the sorted classification information (Z) to evaluate the body temperature regulating function of the target.

As described above, the first threshold value set for the autonomic index and/or the second threshold value set for the perspiration index are/is set for each of different temperatures. As a result, the body temperature regulating function of the target can be more accurately evaluated.

(5) The first and second threshold values may be set based on the environment index representing the environment where the target is situated immediately before the target is measured by the first and second sensors (100) and (200) (immediately before the body temperature regulating function is evaluated). The environment index indicates an index that affects the human cold/hot sensation, and indicates, for example, temperature, humidity, and/or wind velocity.

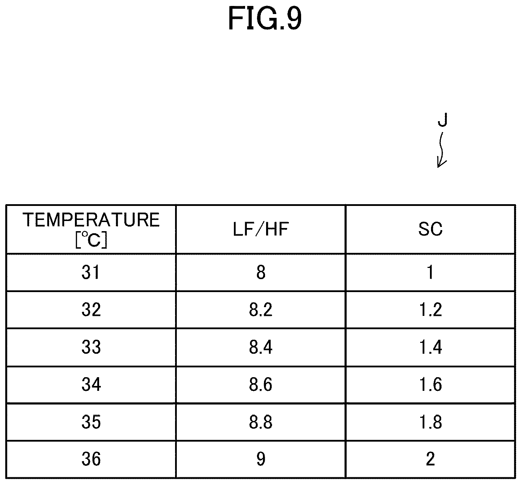

An example device configuration for setting the first and second threshold values based on the environment index will be described with reference to FIGS. 1 and 9. FIG. 9 shows environment-associated information (J) indicating association among the environment index, the first threshold value, and the second threshold value.

In this embodiment, the temperature (the temperature in the environment where the target is situated immediately before the body temperature regulating function is evaluated) serving as a first example of the environment index, the first threshold value, and the second threshold value are associated with one another in the environment-associated information (J).

In this embodiment, in the environment-associated information (J), as the temperature serving as the first example of the environment index increases, each of the first and second threshold values increases.

As illustrated in FIG. 1, the evaluation system (10) further includes a detector (not shown) that detects the environment index. In this embodiment, the detector includes a temperature sensor. The storage (340) stores the environment-associated information (J). The temperature sensor serving as the detector detects the temperature in the environment where the target is situated immediately before the body temperature regulating function is evaluated. Acquiring the detection result of the detector (the temperature in the environment where the target is situated immediately before the body temperature regulating function is evaluated), the evaluation unit (350) selects the first and second threshold values associated with the acquired temperature in the environment-associated information (J). For example, if the detection result of the detector is 34°C, "8.6" is selected as the heart rate variability index (LF/HF), and "1.6" is selected as the skin conductance change (SC). The evaluation unit (350) uses the selected first threshold value to sort the values of the heart rate variability index (LF/HF) in the classification information (Z) (see FIG. 4) into a range of larger values and a range of smaller values, uses the selected second threshold value to sort the values of the skin conductance change (SC) in the classification information (Z) into a range of larger values and a range of smaller values, and uses the sorted classification information (Z) to evaluate the body temperature regulating function of the target. As a result, the body temperature regulating function of the target can be more accurately evaluated.

(6) The first and second threshold values may be set in consideration of the characteristics of the target about the air temperature. A procedure for setting the first and second threshold values will be hereinafter described.

The heart rate variability index (LF/HF) and the skin conductance change (SC) of the target are acquired while the temperature at the location where the target is present is room temperature and the target is at rest. The heart rate variability index (LF/HF) and the skin conductance change (SC) acquired in such a state (the state where the air temperature is room temperature and the target is at rest) reflect the characteristicss of the target about the air temperature. Then, the first and second threshold values are set based on the acquired heart rate variability index (LF/HF) and skin conductance change (SC). For example, a value that is α times as great as the acquired heart rate variability index (LF/HF) is set for the first threshold value, and a value that is β times as great as the acquired skin conductance change (SC) is set for the second threshold value. The values α and β are positive real numbers. The values α and β are fixed values. The value α is, for example, 1.2 (α = 1.2), and the value β is, for example, 3 (β = 3).

(7) The target or any other person may input the first and second threshold values to the evaluation device (300) to set the first and second threshold values. As illustrated in FIG. 1, in this case, the evaluation device (300) includes an input unit (a touch panel, a keyboard, a mouse, or any other component) (not shown) that accepts input of the first and second threshold values. In this case, the first and second threshold values that have already been set may be modified (adjusted) through the input unit so as to be able to be reset.

(8) A variation of the second process (see step S60) shown in FIG. 6 will be described. In the second embodiment, if, in step S30, the evaluation unit (350) (see FIG. 1) evaluates that the body temperature regulating function of the target corresponds to the pattern (D), the second process is performed. However, the present invention is not limited to this.

If, in step S30, the evaluation unit (350) (see FIG. 1) evaluates that the body temperature regulating function of the target corresponds to the pattern (D) (the second pattern), and the temperature detected by the temperature sensor (411) is higher than or equal to a predetermined uncomfortable temperature (e.g., 30°C or higher), the control unit (413) of the air processing device (400) may perform the second process. The predetermined uncomfortable temperature indicates a temperature at which the target may feel uncomfortable. The predetermined uncomfortable temperature indicates a temperature higher than the first temperature (comfortable temperature).

For example, if the indoor temperature is room temperature (e.g., 26°C), and the target is concentrating on his/her work without feeling particular stress from the indoor temperature (i.e., in a state where the amount of his/her perspiration is small), the autonomic index (heart rate variability index (LF/HF)) may increase in a state where the perspiration index (skin conductance change (SC)) is small. In this case, if the body temperature regulating function of the target is evaluated to correspond to the pattern (D), and the air processing device (400) performs the second process, wind at the second temperature (cold temperature) is sent into the room (see FIG. 8) while the target does not feel particular stress from the indoor temperature. Thus, the target may feel stress from the cold wind.

To address this problem, as described above, if the body temperature regulating function of the target is evaluated to correspond to the pattern (D), and the temperature detected by the temperature sensor (411) is higher than or equal to the predetermined uncomfortable temperature, the second process is performed. This configuration can prevent the second process from being performed if, while the indoor temperature is room temperature (lower than the predetermined uncomfortable temperature), the target is concentrating on his/her work (if, while the autonomic index is great, the target does not feel particular stress from the indoor temperature).

As described above, if the evaluation unit (350) (see FIG. 1) evaluates that the body temperature regulating function of the target corresponds to the pattern (D), and the temperature detected by the temperature sensor (411) is lower than the predetermined uncomfortable temperature, the process shown in step S40 (the process of continuing the present control), for example, is performed.

(9) In each of the first and second embodiments, the single first threshold value V (see FIG. 2) and the single second threshold value W (see FIG. 3) are set in the classification information (Z) shown in FIG. 4 to classify the result of evaluating the body temperature regulating function into four (2 × 2) patterns (patterns (A) to (D)). However, the present invention is not limited to this. A plurality of first threshold values and/or a plurality of second threshold values may be set to calssify the result of evaluating the body temperature regulating function into more than four patterns. For example, setting two first threshold values allows the values of the heart rate variability index (LF/HF) to be sorted into three ranges, i.e., a range of larger values, a range of intermediate values, and a range of smaller values; and setting two second threshold values allows the values of the skin conductance change (SC) to be sorted into three ranges, i.e., a range of larger values, a range of intermediate values, and a range of smaller values. Thus, the result of evaluating the body temperature regulating function may be classified into nine (3 × 3) patterns.

(10) The evaluation device (300) may further include a configuration of an evaluation device (300A) to be described later (a configuration for evaluating the exercise performance of the target).

### -Third Embodiment-

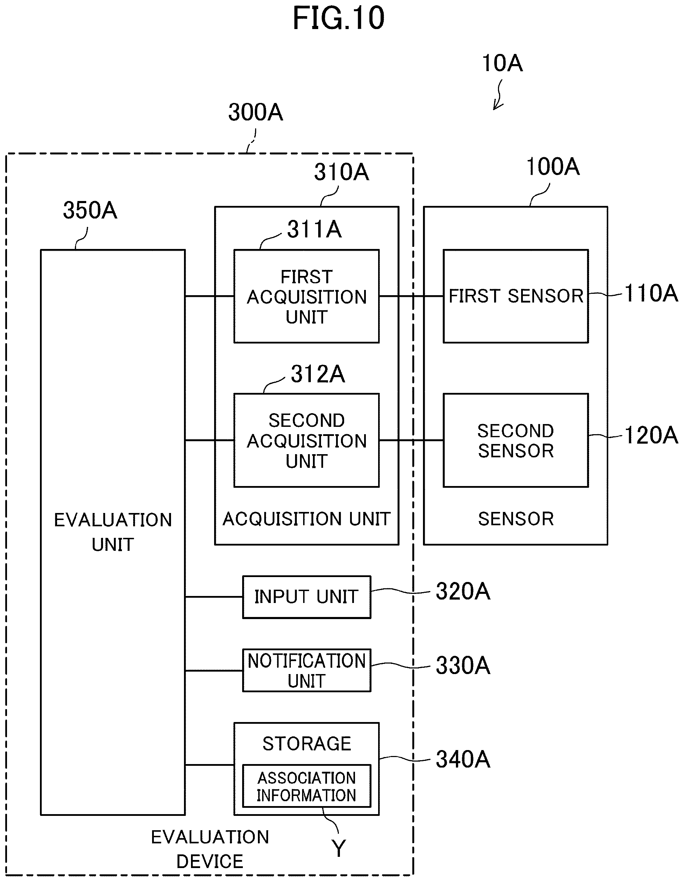

A system (10A) for evaluating exercise performance according to a third embodiment of the present invention will be described with reference to FIG. 10. FIG. 10 is a block diagram illustrating a configuration of the system (10A) for evaluating the exercise performance according to the third embodiment of the present invention. The system (10A) for evaluating the exercise performance may be hereinafter referred to as the "evaluation system (10A)."

### -General Configuration-

As illustrated in FIG. 10, the evaluation system (10A) includes a sensor (100A) and a device (300A) for evaluating the exercise performance. The device (300A) for evaluating the exercise performance may be hereinafter referred to as the "evaluation device (300A)." The exercise performance indicates the degree of the capability of exercise to be performed.

The sensor (100A) is worn by a target to acquire an index of fatigue of the thermoregulation function indicating the degree of fatigue of the body temperature regulating function of the target. The body temperature regulating function indicates the function of maintaining the body temperature through heat dissipation (e.g., perspiration) or thermogenesis (e.g., body shivering) as appropriate to address heat stress. The heat stress indicates the load (e.g., warm and cold stimuli that hinder maintenance of the body temperature) that a human receives from the thermal environment. The degree of fatigue of the body temperature regulating function is defined by the degree of decrease in the frequency at which an action for thermoregulation, such as an action of perspiration, an action of body shivering, or an action of dilating or constricting the blood vessels, is performed in response to an instruction related to the thermoregulation function from the center to the terminals of the autonomic nerve.

In the third embodiment, the index of fatigue of the thermoregulation function indicating the degree of fatigue of the body temperature regulating function of the target includes the heart rate variability index (LF/HF) and the skin conductance change (SC).

The sensor (100A) includes a first sensor (110A) and a second sensor (120A).

The first sensor (110A) has a function that is the same as, or similar to, that of the first sensor (100) (see FIG. 1).

The second sensor (120A) has a function that is the same as, or similar to, that of the second sensor (200) (see FIG. 1).

The evaluation device (300A) includes, for example, a terminal, such as a smartphone or a personal computer (PC). The evaluation device (300A) evaluates the exercise performance of the target using the index of fatigue of the thermoregulation function of the target to output an exercise performance index indicating the exercise performance of the target.

The evaluation device (300) includes an acquisition unit (310A), an input unit (320A), a notification unit (330A), a storage (340A), and an evaluation unit (350A).

The acquisition unit (310A) includes a device for communicating with the sensor (100A). The acquisition unit (310A) is communicably connected to the sensor (100A) in a wired or wireless manner. The acquisition unit (310A) includes, for example, at least one of a device for performing wireless communication (e.g., Bluetooth (registered trademark) or wireless fidelity (Wi-Fi) (registered trademark)) (such as a wireless LAN module) or a device for performing wired communication (such as a communication port connected to a communication cable).

In the third embodiment, the acquisition unit (310A) includes a first acquisition unit (311A) and a second acquisition unit (312A).

The first acquisition unit (311A) has a function that is the same as, or similar to, that of the first acquisition unit (310) (see FIG. 1).

The second acquisition unit (312A) has a function that is the same as, or similar to, that of the second acquisition unit (320) (see FIG. 1).

The input unit (320A) accepts an external instruction to the evaluation device (300A). The input unit (320A) includes, for example, a keyboard, a mouse, and a touch panel.

The notification unit (330A) makes notification of predetermined information. The notification unit (330A) includes, for example, a speaker that emits notification sounds, a display that displays predetermined information, and/or a communication device that transmits predetermined information to an external terminal (such as a smartphone). The predetermined information will be described later.

The storage (340A) includes a main memory, such as a flash memory, a ROM, or a RAM, and may further include an auxiliary memory, a SD memory card, or a USB. The storage (340A) stores various computer programs executed by processors of the evaluation unit (350A) and the acquisition unit (310A).

The storage (340A) stores association information (Y). The association information (Y) will be described later.

The evaluation unit (350A) includes the processor, such as a CPU or a MPU. The evaluation unit (350A) executes a computer program stored in the storage (340A) so as to control elements of the evaluation device (300A).

As shown in FIGS. 2 and 3, during later ones of the high-temperature periods from time t1 to time t2, from time t3 to time t4, from time t5 to time t6, and from time t7 to time t8, exhaustion of the target causes the heart rate variability index (LF/HF) to be greater than during earlier ones of the high-temperature periods. However, the skin conductance change (SC) decreases such that the body temperature cannot be successfully regulated, resulting in an increase in the degree of fatigue of the body temperature regulating function.

Based on the test result shown in FIGS. 2 and 3, the inventors of this application have developed a technique for evaluating the fatigued state of the thermoregulation function (the state of the thermoregulation function), such as the disturbance of the autonomic balance related to the temperature regulating function or deterioration in the peripheral perspiration function, step by step based on the heart rate variability index (LF/HF) and the state of the skin conductance change serving as the perspiration index. The fatigued state is caused by long-time or repetitive exposure to heat load.

In the third embodiment, the inventors of this application have hypothesized that the exercise performance can be evaluated based on the patterns (A) to (D) of the index of fatigue of the body temperature regulating function indicating the degree of fatigue of the body temperature regulating function in the classification information (Z) (see FIG. 4). A method for evaluating the exercise performance based on the hypothesis will be hereinafter described.

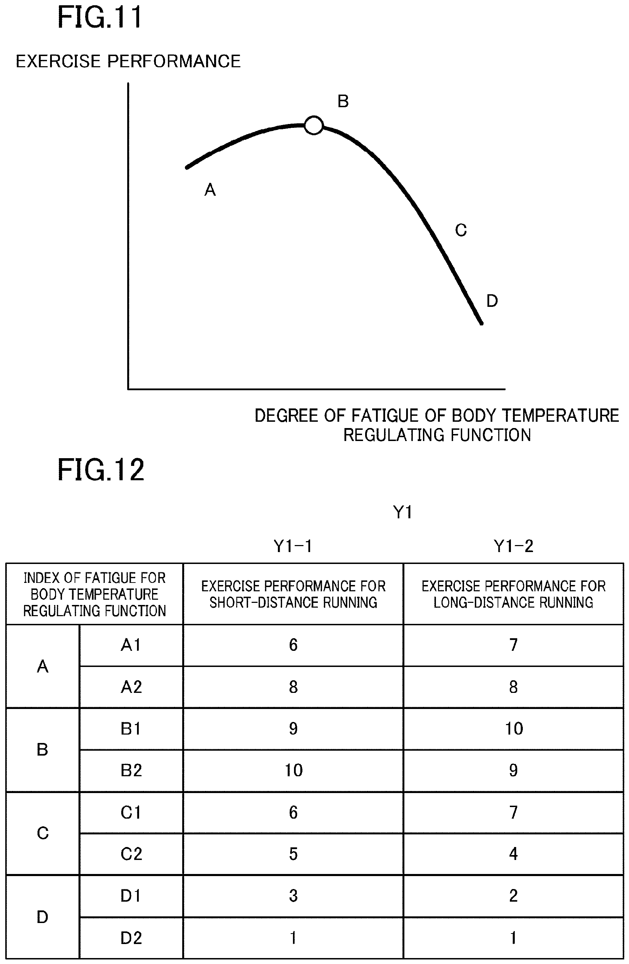

FIG. 11 is a graph showing a hypothesis model of the relation between the index of fatigue of the body temperature regulating function and the exercise performance. The index of fatigue of the body temperature regulating function is expected to increase in the order of the patterns (A), (B), (C), and (D), and the level of the exercise performance is expected to decrease in the order of the patterns (B), (A), (C), and (D).

As shown in FIGS. 4 and 11, if the index of fatigue of the body temperature regulating function corresponds to the pattern (A), the load imposed on the thermoregulation is low and the thermoregulation function has not been fatigued. Thus, the target is estimated to be resting at a neutral temperature and to have a body temperature that is lower than a temperature suitable for exercise. In this case, the target is not fatigued, but has an unsuitable body temperature. Thus, his/her exercise performance is expected to be lower than his/her best exercise performance but to be somewhat high.

If the index of fatigue of the body temperature regulating function corresponds to the pattern (B), the load imposed on the thermoregulation is high and the fatigue of the thermoregulation function is low such that energy is still left over. Thus, in general, a mild exercise, such as a warm-up exercise, is estimated to allow the target to have a body temperature that is suitable for excercise. In this case, the target still has energy left over, and has a body temperature that is suitable for exercise. Thus, his/her exercise performance is expected to be the best.

If the index of fatigue of the body temperature regulating function corresponds to the pattern (C), the load imposed on the thermoregulation is high and the fatigue of the thermoregulation function is moderate such that a small amount of energy is left over. Thus, an estimation is made that continuing exercise will allow the target to have his/her body temperature increased to the body temperature that is suitable for exercise, and will increase the load imposed on the central nervous system that is responsible for thermoregulation to address the increase in body temperature and the terminal function that acts on an actual heat dissipation phenomenon. In this case, the target has a body temperature that is suitable for exercise, but does not have energy left over for the thermoregulation function. Thus, his/her exercise performance is expected to be lower than if the index of fatigue corresponds to the pattern (A).

If the index of fatigue of the body temperature regulating function corresponds to the pattern (D), the load imposed on the thermoregulation is high, and the fatigue of the thermoregulation function is high such that the body temperature is hardly maintained. Thus, an estimation is made that the thermoregulation function will be exhausted to reduce the amount of perspiration, and that continuing exercise will prevent an increase in the body temperature from being adequately reduced. In this case, degrading the exercise performance may reduce an increase in the body temperature. Thus, the exercise performance is expected to be lower than if the index of fatigue corresponds to the pattern (C).

Based on the above-described hypothesis, the inventors of this application have modeled the relation between the index of fatigue of the body temperature regulating function and the exercise performance. Specifically, if the degree of fatigue of the body temperature regulating function is proper, such as if it corresponds to the pattern (B), the exercise performance is estimated to be high.

The association information (Y) will be described with reference to FIGS. 4 and 12.

As shown in FIG. 4, the degree of fatigue of the thermoregulation function is associated with the amount of perspiration and the autonomic balance, and the index of fatigue of the thermoregulation function is the lowest in the pattern (A), and increases in the order of the patterns (B), (C), and (D). Each of the patterns (A) to (D) may be divided into a plurality of sub-items, such as patterns (A1) and (A2) for the pattern (A), patterns (B1) and (B2) for the pattern (B), patterns (C1) and (C2) for the pattern (C), and patterns (D1) and (D2) for the pattern (D) (see FIG. 12), depending on the magnitudes of the amount of perspiration and the autonomic balance within the range of the pattern. As shown in FIG. 12, the association information (Y) indicates information indicating association of different indexes of exercise performance with different indexes of fatigue of the thermoregulation function. In the third embodiment, the association information (Y) includes the first association information (Y1). The first association information (Y1) includes information (first-type association information) (Y1-1) associated with a first type of exercise and information (second-type association information) (Y1-2) associated with a second type of exercise.

In the third embodiment, the index of exercise performance is represented by a numerical value of 1 to 10. The exercise performance is defined to increase as the numerical value representing the index of exercise performance increases.

FIG. 12 shows the first association information (Y1).

As shown in FIG. 4, in the third embodiment, the index of fatigue of the thermoregulation function includes the heart rate variability index (LF/HF) and the skin conductance change (SC).

The information (Y1-1) associated with the first type of exercise and the information (Y1-2) associated with the second type of exercise indicate association between the index of fatigue of the thermoregulation and the exercise performance.

In the third embodiment, the first threshold value V (see FIG. 2) is set to a value of nine. In FIG. 4, within the range where the heart rate variability index (LF/HF) is less than nine, the heart rate variability index (LF/HF) is set to be "small," and within the range where it is greater than or equal to nine, it is set to be "large."

In the third embodiment, the second threshold value W (see FIG. 3) is set to a value of two. In FIG. 4, within the range where the skin conductance change (SC) is less than two, the skin conductance change (SC) is set to be "small," and within the range where it is greater than or equal to two, it is set to be "large."

The numerical values described in the information (Y1-1) associated with the first type of exercise and the information (Y1-2) associated with the second type of exercise each indicate the associated index of the exercise performance observed if the index of fatigue of the body temperature regulating function corresponds to any one of the patterns (A), (B), (C), and (D) (see FIG. 12).

In the third embodiment, the first association information (Y1) includes a plurality of types of information (Y1-1, Y1-2) set according to the types of exercise. The information (Y1-1) associated with the first type of exercise is intended for short-distance running, and is associated with the exercise performance for short-distance running. The information (Y1-1) associated with the first type of exercise is used to output the exercise performance for short-distance running. The information (Y1-2) associated with the second type of exercise is intended for long-distance running, and is associated with long-distance running. The information (Y1-2) associated with the second type of exercise is used to output the exercise performance for long-distance running.

The plurality of types of association information are set by previously measuring the relation between the index of fatigue of the thermoregulation function and the exercise performance or the work performance. In each of the plurality of types of association information (the information (Y1-1) associated with the first type of exercise and the information (Y1-2) associated with the second type of exercise), the magnitude of the index of the exercise performance associated with the index of fatigue of the thermoregulation function is set so as to be adapted to the type of the intended exercise. For example, short-distance running requires higher explosive power than long-distance running. Thus, the muscle temperature needs to be high to increase the muscle contractile force. Thus, the index of the exercise performance in each of the information (Y1-1) associated with the first type of exercise and the information (Y1-2) associated with the second type of exercise is set so that the degree of fatigue of the thermoregulation function observed when the index of the exercise performance reaches its peak value "10" is higher for short-distance running than for long-distance running. In the third embodiment, in the case of short-distance running, when the degree of fatigue of the thermoregulation function corresponds to the sub-pattern (B2), the index of the exercise performance is set to reach its peak value "10" as indicated by the information (Y1-1) associated with the first type of exercise. In the case of long-distance running, when the degree of fatigue of the thermoregulation function corresponds to the sub-pattern (B1), the index of the exercise performance is set to reach its peak value "10" as indicated by the information (Y1-2) associated with the second type of exercise.

### -Example of Processing of Evaluation Unit (350A) -

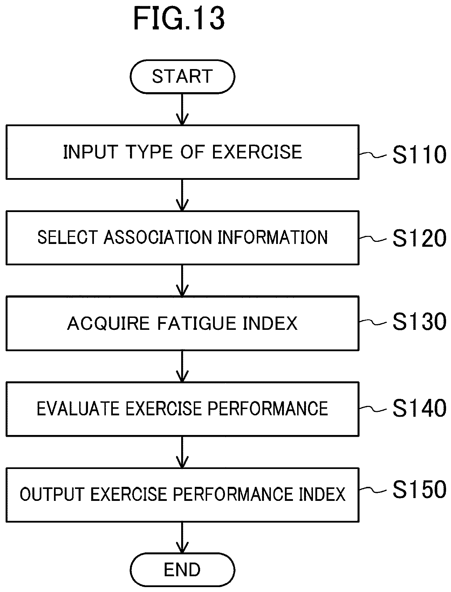

An example of processing of the evaluation unit (350A) will be described with reference to FIGS. 10, 12, and 13. FIG. 13 is a flowchart showing an example of processing of the evaluation unit (350A).

As shown in FIGS. 10, 12, and 13, in step S110, information indicating the type of exercise to be performed by the target is input from the input unit (320A).

In step S120, the evaluation unit (350A) selects information associated with the exercise input from the input unit (320A) from among the plurality of types of information (the information (Y1-1) and the information (Y1-2)). For example, if information indicating short-distance running is input from the input unit (320A) in step S110, then the evaluation unit (350A) selects the second association information (Y1-1) associated with short-distance running in step S120.

In step S130, the processor of the acquisition unit (310A) determines the index of fatigue of the thermoregulation function of the target using the various types of information acquired by the first and second acquisition units (311A) and (312A). As a result, the acquisition unit (310A) acquires the index of fatigue of the thermoregulation function of the target. In the third embodiment, any one of the patterns (A1) to (D2) (see FIG. 12) is acquired as the index of fatigue of the thermoregulation function of the target.

In step S140, the evaluation unit (350A) evaluates the exercise performance of the target by using the information selected in step S120 and the index of fatigue of the thermoregulation function of the target acquired in step S130 (derived from the heart rate variability index (LF/HF) and the skin conductance change (SC)).

For example, if the information selected in step S120 is the information (Y1-2), and the index of fatigue of the body temperature regulating function of the target acquired in step S130 falls under the pattern (B2), the value "9" associated with the pattern (B2) in the information (Y1-2) is determined to be the index of the exercise performance of the target, thereby evaluating the exercise performance of the target.

In step S150, the evaluation unit (350A) makes the notification unit (330A) output information indicating that the index of the exercise performance of the target determined in step S140 is 9. As a result, the target can check the index of his/her own exercise performance.

When the process shown in step S 150 ends, the process ends.

### -Advantages of Third Embodiment-

As described above with reference to FIGS. 2 to 4 and 10 to 13, the evaluation unit (350A) can evaluate the exercise performance of the target using the heart rate variability index (LF/HF) and the skin conductance change (SC) that form the index related to the thermoregulation function of the target.

In addition, a plurality of types of the association information (Y1) are set according to the types of exercise. This allows the exercise performance of the target to be effectively evaluated so as to be adapted to the exercise to be performed by the target.

### -Fourth Embodiment-

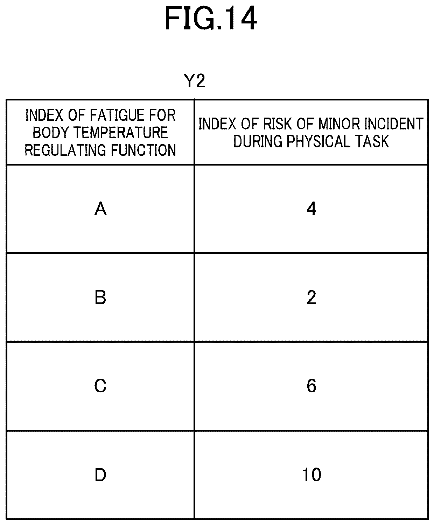

A fourth embodiment of the present invention will be described with reference to FIGS. 10, 4, and 14. FIG. 14 shows second association information (Y2) that is a second example of association information (Y).

As shown in FIGS. 10 and 14, in the fourth embodiment, the association information (Y) stored in the storage (340A) is configured as the second association information (Y2).

The second association information (Y2) indicates association of different indexes of exercise performance with different indexes of fatigue of the thermoregulation function. In the second association information (Y2), the degree of fatigue of the thermoregulation function is represented by A to D.

A first example of a procedure for determining the indexes (A) to (D) of fatigue of the thermoregulation function in the second association information (Y2) will be described. The index of risk of a minor incident, which is an example of the index of fatigue of the thermoregulation function, is represented by a numerical value of one to ten, and indicates that the higher the numerical value is, the higher the risk of occurrence of a minor incident during a physical task is.

In the first example, a sensor (100A) is configured as a temperature sensor. The index of fatigue of the thermoregulation function is determined by a processor provided for an acquisition unit (310A), based on the pheripheral skin temperature and deep-body temperature of the target acquired from the temperature sensor by the acquisition unit (3 10A). As a result, the acquisition unit (3 10A) acquires the index of fatigue of the thermoregulation function.

In this case, for example, if the peripheral skin temperature is lower than 34°C, and the deep-body temperature is lower than 37.5°C, the index of fatigue corresponds to the pattern (B), and the index of risk of a minor incident during a physical task is determined to be four. If the peripheral skin temperature is higher than or equal to 34°C and lower than 35°C, and the deep-body temperature is lower than 37.5°C, the index of fatigue corresponds to the pattern (B), and the index of risk of a minor incident during a physical task is determined to be two. If the peripheral skin temperature is higher than or equal to 35°C, and the deep-body temperature is lower than 37.5°C, the index of fatigue corresponds to the pattern (C), and the index of risk of a minor incident during a physical task is determined to be six. If the peripheral skin temperature is higher than or equal to 35°C, and the deep-body temperature is higher than or equal to 37.5°C, the index of fatigue corresponds to the pattern (D), and the index of risk of a minor incident during a physical task is determined to be ten.

A second example of the procedure for determining the indexes (A) to (D) of fatigue in the second association information (Y2) will be described.

In the second example, a sensor (100A) includes a temperature sensor and a timer. The temperature sensor detects the air temperature in the environment where the target is situated immediately before the evaluation unit (350A) evaluates the exercise performance of the target (environment temperature). The timer measures the time (stay time) during which the target is situated in the environment. The acquisition unit (310A) acquires information indicating the environment temperature detected by the temperature sensor and information indicating the stay time measured by the timer. Then, the processor provided for the acquisition unit (310A) determines the index of fatigue of the thermoregulation function, based on the environment temperature and the stay time.

In this case, for example, if the environment temperature is a predetermined comfortable temperature (the temperature at which a human generally feels comfortable), the index of fatigue of the thermoregulation function is determined to be smaller (e.g., any one of (A) to (C)). If the environment temperature is a temperature deviating from the predetermined comfortable temperature (the temperature at which a human generally feels too cold or too hot), the index of fatigue of the thermoregulation function is determined to increase with increasing stay time.

If the association information (Y) is configured as the second association information (Y2), the evaluation unit (350A) outputs the risk of a minor incident during a physical task associated with the index of fatigue of the thermoregulation function in the second association information (Y2) as the result of evaluating the risk of a minor incident during a physical task of the target.

In the fourth embodiment, a plurality of types of first association information (Y1) are not set according to the types of exercise unlike the third embodiment, and the association information (Y) is configured as one type of second association information (Y2) that does not specify the type of exercise. In this case, when, in the flowchart shown in FIG. 13, the process of evaluating the exercise performance of the target is performed, the need for the process shown in step S 110 and the process shown in step S 120 is eliminated. As a result, a process for evaluating the exercise performance of the target can be quickly performed. However, the present invention is not limited to this. Also in the fourth embodiment, a plurality of types of second association information (Y2) may be set according to the types of exercise. For example, second association information (Y2) for light work in a factory and second association information (Y2) for outdoor road construction may be set.

In the third embodiment, the first association information (Y1) is configured as two types of information such as information (Y1-1) for short-distance running and information (Y1-2) for long-distance running, but may be configured as one type of information that does not specify the type of exercise or work just like the second association information (Y2) of the fourth embodiment.

### -Advantages of Fourth Embodiment-

As described above with reference to FIGS. 10, 4, and 14, the evaluation unit (350A) can evaluate the risk of a minor incident during a physical task of the target by using the degree of fatigue serving as an index related to the thermoregulation function of the target.

### -Fifth Embodiment-

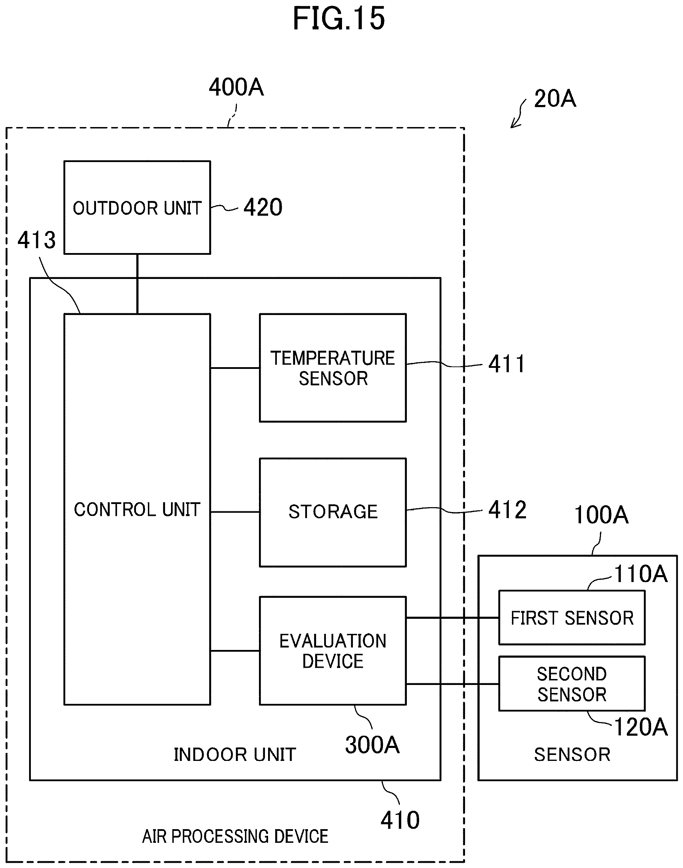

An evaluation system (20A) according to a fifth embodiment of the present invention will be described with reference to FIG. 15. FIG. 15 is a block diagram illustrating a configuration of the evaluation system (20A) according to the fifth embodiment of the present invention.

### -General Configuration-

As illustrated in FIG. 15, the evaluation system (20A) includes a sensor (100A) and an air processing device (400A).

An air processing device (400A) includes an evaluation device (300A) unlike the air processing device (400) of the second embodiment (see FIG. 5), and other configurations thereof are the same as, or similar to, those of the air processing device (400) of the second embodiment.

In the fifth embodiment, the evaluation device (300A) is an electronic component incorporated into or externally attached to a housing of an indoor unit (410). However, the present invention is not limited to this. The evaluation device (300A) may be incorporated into a bracelet device including a first sensor and a second sensor, and may output the evaluation result to a communication unit incorporated into the indoor unit to make a control unit acquire the evaluation result.

### -Example of Operation of Air Processing Device (400A)-

An example of an operation of the air processing device (400A) will be described with reference to FIGS. 10 and 15.

As illustrated in FIGS. 10 and 15, the control unit (413) of the air processing device (400A) acquires the index of exercise performance of the target output from the evaluation device (300). Then, if the index of exercise performance of the target acquired from the evaluation device (300A) is less than a predetermined target value, the control unit (413) of the air processing device (400A) performs the process of changing the set temperature of the air processing device (400A) until the index of exercise performance acquired from the evaluation device (300A) becomes greater than or equal to the predetermined target value. The air processing device (400A) may be configured to perform the process of changing the set temperature, set humidity, and/or airflow of the air processing device (400A) until the index of exercise performance acquired from the evaluation device (300A) becomes greater than or equal to the predetermined target value. Thus, the temperature, humidity, and/or airflow in the space where the target is present are/is controlled. A temperature processing device may be configured to control the temperature, humidity, and/or airflow of an appliance or clothing touched by the target. The temperature processing device includes, for example, a fan provided for air-conditioning clothes.

### -Advantages of Fifth Embodiment-

As described above with reference to FIGS. 10 and 15, the air processing device (400A) includes the evaluation device (300A). This allows an action of the air processing device (400A) to be controlled in consideration of the result obtained by the evaluation device (300A) evaluating the exercise performance of the target.

### <<Other Embodiments>>

While the third to fifth embodiments have been described above, it will be understood that various changes in form and details may be made without departing from the spirit and scope of the claims (e.g., (11) to (12)). The embodiments and the variations thereof may be combined and replaced with each other without deteriorating intended functions of the present disclosure.

(11) In each of the third to fifth embodiments, the heart rate variability index (LF/HF) used to grasp how the heart rate works is used as the autonomic index. However, the present invention is not limited to this. For example, the pulse rate variability index used to grasp how the pulse rate works may be used as the autonomic index. In this case, the first sensor (110A) includes a pulse wave sensor.

(12) If, in step S150 shown in FIG. 13, the index of exercise performance of the target is output, the notification unit (330A) may be configured to make notification of information related to the index of exercise performance of the target. As a result, the target can check his/her own exercise performance.

An example of an action of the notification unit (330A) will now be described.

The acquisition unit (310A) successively acquiring the indexes of fatigue of the thermoregulation function of the target allows the evaluation unit (350A) to successively output the indexes of exercise performance of the target. If, in this state, the index of exercise performance of the target is greater than or equal to a predetermined value, the notification unit (330A) makes notification of predetermined information related to an action of the target. The predetermined value indicates, for example, a somewhat great index of exercise performance (e.g., nine or more) that allows effective exercise. The predetermined information indicates, for example, that a situation where the index of exercise performance of the target is greater than or equal to the predetermined value shows that the target has performed an adequate warm-up and that it is time to stop the warm-up. As a result, the target checking the predetermined information of which notification is made by the notification unit (330A) can recognize the time to stop his/her own warm-up. The target recognizing the time to stop his/her own warm-up can exercise with his/her own exercise performance enhanced. Furthermore, if the degree of fatigue of the thermoregulation function deteriorates, and a decrease in exercise performance is detected, recommending a break or rehydration can prevent the target from exercising with his/her performance decreased. Applying such a technique not only to the exercise performance during an athletic competition but also to the work efficiency of a physical task, such as road construction, can improve the operator's work efficiency.

### INDUSTRIAL APPLICABILITY

As can be seen from the foregoing description, the present disclosure is useful for a device for evaluating a body temperature regulating function, an air processing device, and a method for evaluating a body temperature regulating function.

### DESCRIPTION OF REFERENCE CHARACTERS

- 10: System For Evaluating Body Temperature Regulating Function
- 300: Device For Evaluating Body Temperature Regulating Function
- 310: First Acquisition Unit
- 320: Second Acquisition Unit
- 330: Notification Unit
- 340: Storage
- 350: Evaluation Unit
- 400: Air Processing Device
- Z: Classification Information

## Claims

1. A method for evaluating a body temperature regulating function, the method comprising:
acquiring an autonomic index of a target;
acquiring a perspiration index of the target; and
evaluating the body temperature regulating function of the target by using the autonomic index of the target and the perspiration index of the target.

2. The method of claim 1, wherein
the autonomic index includes at least one of a heart rate variability index or a pulse rate variability index, and
the perspiration index includes at least one of a skin conductance change or an amount of perspiration.

3. The method of claim 1 or 2, wherein
in the evaluating, the body temperature regulating function of the target is evaluated by further using classification information (Z) obtained by classifying a result of evaluating the body temperature regulating function into a plurality of patterns based on a threshold value set for each of the autonomic index and the perspiration index.

4. The method of claim 3, wherein
the plurality of patterns are associated with a plurality of index sets, respectively,
each of the plurality of index sets is configured as a combination of the autonomic index and the perspiration index each having a range defined by the threshold value, and
in the evaluating, one of the plurality of index sets having a range including the autonomic index of the target and the perspiration index of the target is selected, and one of the plurality of patterns associated with the selected index set is evaluated to correspond to the body temperature regulating function of the target.

5. The method of claim 3 or 4, wherein
the threshold value or values set for the autonomic index and/or the perspiration index is/are set for each of different temperatures.

6. The method of claim 3 or 4, wherein
the threshold value set for each of the autonomic index and the perspiration index is set based on an environment index that represents an environment where the target is situated immediately before the body temperature regulating function is evaluated.

7. The method of claim 3 or 4, wherein
the threshold value set for each of the autonomic index and the perspiration index is set in consideration of characteristics of the target about an air temperature.

8. The method of any one of claims 3 to 7 further comprising:
making notification of predetermined information to outside if a pattern of the target evaluated in the evaluating corresponds to a predetermined one of the plurality of patterns, and a state where the target corresponds to the predetermined pattern has continued for a predetermined period.

9. The method of any one of claims 1 to 8 further comprising:
controlling a temperature in a room where the target is present, through an air processing device (400) based on an evaluation result in the evaluating.

10. The method of claim 9, wherein
the controlling includes:
making the air processing device (400) operate so that the temperature in the room is equal to a predetermined first temperature, if the body temperature regulating function of the target is evaluated to fall under a first pattern; and
making the air processing device (400) operate so that the temperature in the room decreases to a predetermined second temperature lower than the predetermined first temperature and then increases to the predetermined first temperature, if the body temperature regulating function of the target is evaluated to fall under a second pattern that is different from the first pattern.

11. The method of claim 9, wherein
the controlling includes:
making the air processing device (400) operate so that the temperature in the room is equal to a predetermined first temperature, if the body temperature regulating function of the target is evaluated to fall under a first pattern; and
making the air processing device (400) operate so that the temperature in the room decreases to a predetermined second temperature lower than the predetermined first temperature and then increases to the predetermined first temperature, if the body temperature regulating function of the target is evaluated to fall under a second pattern that is different from the first pattern and the temperature in the room is higher than or equal to a predetermined temperature.

12. The method of any one of claims 1 to 11 further comprising:
acquiring an index of fatigue of a thermoregulation function indicating a degree of fatigue of the body temperature regulating function of the target; and
evaluating exercise performance of the target using the index of fatigue of the thermoregulation function of the target.

13. The method of claim 12, wherein
in the evaluating of the exercise performance, evaluating the exercise performance of the target allows an exercise performance index indicating the exercise performance of the target to be output.

14. The method of claim 12 or 13, wherein
the acquiring of the index of fatigue includes:
acquiring of an autonomic index of the target; and
acquiring of a perspiration index of the target.

15. The method of claim 14, wherein
the autonomic index includes a heart rate variability index, and
the perspiration index includes a skin conductance change or an amount of perspiration.

16. The method of any one of claims 12 to 15, wherein
in the evaluating of the exercise performance, the exercise performance of the target is evaluated by further using association information (Y) indicating association between each of different values of the index of fatigue of the thermoregulation function and an exercise performance index indicating the exercise performance.

17. The method of claim 16, wherein
the association information (Y1) includes a plurality of types of information (Y1-1, Y1-2) set according to types of exercise.

18. The method of claim 16, wherein
the association information (Y2) includes a plurality of types of information set according to risks related to the thermoregulation function during an exercise.

19. The method of any one of claims 12 to 18 further comprising:
making notification of information related to a result of evaluating exercise performance of the target.

20. The method of any one of claims 12 to 19 further comprising at least one of:
making an air processing device (400) control a temperature, a humidity, and/or an airflow in a space where the target is present, based on an evaluation result in the evaluating of the exercise performance; or making a temperature processing device control a temperature, a humidity, and/or an airflow of an appliance or clothing touched by the target.

21. A device for evaluating a body temperature regulating function, the device comprising:
a first acquisition unit (310) configured to acquire an autonomic index of a target;
a second acquisition unit (320) configured to acquire a perspiration index; and
an evaluation unit (350) configured to evaluate the body temperature regulating function of the target by using the autonomic index of the target and the perspiration index of the target.

22. An air processing device comprising:
the device for evaluating the body temperature regulating function of claim 21.
